# EUROPEAN PATENT SPECIFICATION

(11) **EP 1 377 218 B1**
(45) Date of publication and mention of the grant of the patent: **24.01.2007**
(21) Application number: 02700191.6
(22) Date of filing: 01.03.2002
(51) Int. Cl.: A61B 7/02

(54) **AN AUDIO INTERFACE SYSTEM FOR MEDICAL USE**
AUDIOINTERFACESYSTEM ZUR MEDIZINISCHEN VERWENDUNG
SYSTEME INTERFACE AUDIO A USAGE MEDICAL

(30) Priority: 01.03.2001 DK 200100336
(43) Date of publication of application: 07.01.2004
(73) Proprietor: Bang & Olufsen Medicom A/S, 7600 Struer (DK)
(72) Inventor: BAEKGAARD, Knud, Erik, DK-7500 Holstebro (DK); ANDERSEN, Bjorn, Knud, DK-7600 Struer (DK)
(74) Representative: Brock-Nannestad, George
(86) International application number: PCT/DK2002/000135
(87) International publication number: WO 2002/069804

(56) References cited:
- WO-A2-98/34542
- US-A- 5 550 902

## Description

The invention relates to an audio interface system for medical use comprising an electronic source for audio signals, and a signal path for audio signals comprising a headset connectable to the ears of a physician or other medical personnel.

The trained ear is used by physicians as one of the most important diagnostic tools, both in conjunction with stethoscopes and unaided. Furthermore the ear is used in person-to-person communication, both with patients and colleagues, and furthermore the ear is used for telephone communication. Much of the input to the ear occurs under adverse conditions, with various types of background noise being present. The ear is efficient in filtering the sound impressions, and may be trained to perceive certain phenomena in almost masking noise environments or to disregard certain types of noise. The qualified physician will have obtained extensive training in the use of a stethoscope, to such a degree that a particular type of stethoscope may be the preference for performing exacting work, for the whole professional life of the physician. The physician's ear must adapt to all the many conditions it encounters, and the stethoscope will be removed for certain types mentioned above and replaced for ausculation. For instance, if the physician is to listen to spectral modifications performed by a signal processing system on a physiological sound - from heart or lungs, he will remove the stethoscope and put on headphones. It is well-known that aural fatigue ensues if the ear is required to strain to obtain relevant information for extended periods of time, and e.g. mobile phone conversations via psychoacoustically data reduced communication links, which interrupt other aural tasks, are known to cause stress conditions affecting concentration and ultimately the ability to distinguish between stimuli.

Much would be gained if the physician were able to keep his ears plugged by the earpieces of the stethoscope, because that would filter out most extraneous noises, and in particular high frequency noises. However, the chestpiece is a poor substitute for the unaided ears as a receiver for airborne sound, and communication with others would have to be on a "speaking tube" basis, which is entirely impractical. Also, it might be desirable to move the chestpiece to the receiver of a telephone when speaking on the telephone while wearing a stethoscope, however the spectral imbalance between the received voice and the mixture of bone-conducted own voice with telephone receiver reproduction of the same own voice causes an unnatural communication. However, there are many situations where it would be relevant to be able to perform a natural conversation during ausculation, but this is currently inhibited by bone conduction of the physician's own voice which is perceived as a loud representation of the own voice with heavy bass emphasis

The issue becomes relevant for example when the physician is carrying out auscultation on a patient and needs to analyse the sound further, e.g. by use of an algorithm on his personal computer. In order to do so the physician needs to record the auscultation signal with an electronic stethoscope, then transfer the signal to the personal computer, then analyse the signal, and finally transfer the feature extracted signal back to the stethoscope for proper acoustic reproduction. During the analysis the physician may need additional patient related information and he therefore interviews the patient, however he desires not to remove the stethoscope during this interview as the final listening session requires its presence.

Prior art techniques have not been helpful in obtaining the advantages of using the stethoscope as a noise-reducing sound transducer for all the necessary use of the ear in the physician's professional day.

In relation to dedicated healthcare management systems, where the central elements are the recording, archiving and reproduction of e.g. human auscultation sound signals as obtained by use of a stethoscope, there has been demonstrated several interesting solutions. For example, there exist systems that use an analogue stethoscope with electrical signal outlet thereby enabling the registration of the signal on a medium having an analogue electrical interface, e.g. a tape recorder, a mini-disc or possibly on a personal computer using the sound card input facility. Typically however, in order to enable registration and subsequent archiving of interesting sound signals using an analogue system, it would be required to carry around both the stethoscope as well as the registration medium, and the registration would therefore necessarily be synchronous in time and place. A system incorporating a local storage facility, e.g. realised using digital technology would overcome this fundamental problem, as it would allow for immediate local storage thereby facilitating asynchronous signal storage and registration. Here however, it is necessary for the storage to have sufficient capacity: When performing auscultation, e.g. of a person's respiratory- or cardiac function, it is typically necessary to take measurements at a number of different locations on the person's thorax. The storage capacity should consequently allow for a plurality of isolated recordings.

Having captured the measured signal it may be archived e.g. in the patient's file. If the signal is captured digitally or if an analogue signal has been digitised e.g. using the sound card of a personal computer or using equipment with similar functionality, the digital representation of the signal may be filed electronically in an electronic patient file. Several existing systems facilitate digital archiving of the measured auscultation sound signals. Having obtained the digital signal it is appealing to perform advanced digital signal analysis in order to extract specific relevant information from the signal, thereby aiding the physician in his search for a diagnosis. There are examples of healthcare management systems offering signal analysis as an aid to the physician in the process of diagnosing. At some point in the signal analysis it becomes relevant, or even necessary, for the physician to listen to the acoustically reproduced enhanced signal. Even though modern digital signal processing algorithms can be made highly sensitive towards specific acoustic phenomena, such automated algorithms are not likely to exceed the capacity of the human hearing in the sensation of weak abnormalities. The human ear and hearing incorporate the present audible impression as well as previously acquired experience, and furthermore the person's analytical skills, and is therefore a highly sophisticated, sensitive, accurate and unsurpassed instrument in the signal analysis e.g. of recorded auscultation sound signals.

In the acoustic reproduction of the signal to the physician's ears it is essentially important to maintain a high level of quality at least as high as if the signal had been transmitted directly through the originally recording stethoscope. These demands effectively eliminate the usage of the conventional personal computer headset, as the acoustic properties of such are too poor, e.g. the low frequency sensitivity is typically so low that the reproduction of cardiac sound signals would distort completely.

However through reproduction e.g. via the originally recording electronic stethoscope the acoustic qualities would per definition be adequate. Also this has been presented previously, using wire-based connections unifying the personal computer sound card analogue output terminal to e.g. a reproducing stethoscope, where the signal reproduces through the isolated electroacoustic headset. However during reproduction the same problems as experienced during signal registration occurs: The wire-based analogue reproduction system forces synchronicity between the sound signal generation and its acoustic reproduction. Furthermore the reproduction using only the original stethoscope's headset restricts the listener to the use of that specific product regardless of his preferences. In a situation where a physician requires the assistance from e.g. a colleague in narrowing down the proper diagnosis of a patient's disease - a second opinion - he would have to rely on a person who used the same hardware equipment, or alternatively was physically close by. He could transmit the digital domain signal to a physically distant location but the recipient would need to use similar hardware in order to reproduce the acoustic signal as intended. This limitation in the presently existing systems strongly affects the freedom of the physician to use his preferred stethoscope, furthermore it forces him to use the specific stethoscope which exists within the frame of the system or alternatively to use more than one type of stethoscope. If he settles for two types of stethoscope it is highly plausible that his ability to perform state-of-the-art listening to and diagnosing of auscultation sounds, in particular cardiac sound signals, is significantly degraded as his auditory system would need to adapt to two different conditions instead of one only. By settling for two different stethoscopes instead of only one preferred type the physician's professional qualities are therefore potentially degraded.

The reproductive subsystem of the construction described in US 5,812,678 demonstrates a way to accomplish acoustic reproduction of electronically stored signals using a plurality of different stethoscope types. However, it does not provide solutions ensuring that the reproduced acoustic signal maintains the necessary high level of quality, e.g. that the critically important low frequency area of the auscultation signal remains undistorted. The suggested reproductive subsystem in US 5,812,678 connects to the stethoscope through hand-held application of the stethoscope bell front side towards a flat disc having an orifice through which the sound couples from the reproduction subsystem speaker. However, the acoustical seal of the connection between the stethoscope bell and the speaker is vital to the high quality sound transmission as even small leaks will cause the low frequency content of the resulting reproduced acoustic signal to suffer a dramatical level reduction, thereby distorting the resulting signal presented to the physician's ears. Another problem with the necessary hand-held application of the stethoscope bell towards the speaker system demonstrated in US 5,812,678 is that it unnecessarily occupies at least one of the physician's hands leaving him with limited freedom to operate e.g. a personal computer performing online signal analysis on the auscultation signal.

According to the invention it has been found that it is possible to obtain all the advantages of the stethoscope without the problems mentioned above in a general interface system. Such a system is particular in that said electronic source for an audio signal is connected to an electroacoustic transducer which is releasably connected to the chestpiece of a stethoscope in a sealed manner in order that the earpieces of the stethoscope convey the audio signal to the ear.

In an embodiment of the invention the signal path for the audio signals obtained in the chestpiece is constituted by the tubing and earpieces of a conventional stethoscope.

In a further embodiment of the invention the chestpiece is constituted by a microphone providing an input to signal processing means in or in conjunction with the stethoscope which provides an output signal to electroacoustic transducers in conjunction with the earpieces of a stethoscope. This in effect means that the electroacoustic transducer converting the electronic signal from the source is acting upon an electronic stethoscope.

In a further embodiment of the invention the electronic representation of the audio signal has been signal processed in order to compensate for the transfer function of the stethoscope. This means that any impulse transfer function irregularities in a conventional stethoscope are compensated when the stethoscope is used as a part of the link beetween the signal source and the ear. It also means that in case an electronic stethoscope is in a mode which enhances certain frequency bands, the signal is given a suitable frequency dependent pre-emphasis.

In a further embodiment of the invention the signal processing comprises a linearising compensation of the transfer characteristics of the electroacoustic transducer connected to the chestpiece. This means that the effect of the coupling cavity and the resonance of a diaphragm on the chestpiece are also compensated.

In a further embodiment of the invention the signal processing ensuring proper acoustic reproduction characteristics is able to adapt principally to all known stethoscope types regardless of chestpiece geometries. E.g. through customisation of the mechanical properties of the audio interface to allow for assembly with all known stethoscope types each combination has one optimal filtering scheme that is predefined in the system. This feature is extremely valuable for the distribution of the reproduction sound signal to persons who prefer to or need to listen by the aid of a specific stethoscope type, conventional or electronic.

In a further embodiment of the invention the linearising compensation is obtained by feedback via a monitoring microphone mounted in conjunction with the coupling cavity.

In a further embodiment of the invention the electronic source for audio signals is a soundcard connected to a personal computer. This embodiment permits the reception of audio files via data transmission networks and conversion into an audio signal listened to by the physician by means of his stethoscope. This feature is extremely useful for remote discussion (obtaining a "second opinion") of a particular auscultated sound, because the physician originating the sound is able to discuss directly with a colleague about the sound with the assurance that his colleague is obtaining precisely the same acoustical input as himself.

In a further embodiment of the invention the electronic source for audio signals is a receiver for telephone signals.

In a further embodiment of the invention the audio signals are standardised physiological signals for ausculation. This permits training and calibration of the physician by means of signals displaying features which the physician must be able to distinguish and identify, such signals being stored or generated in the digital domain in a personal computer and transferred via the soundcard to the electroacoustic transducer.

In a further embodiment of the invention the audio signals are representations of physiological signals mixed with noise signals obtained from the environment. This permits the training of the phycisian in diverting attention from noises such as pumping noises, whirring noises, noises from compressors and the walking action of surrounding persons.

In a further embodiment of the invention the releasable connection comprises elastic clamping means. These means may be either permanently fitted on the interface unit or on the stethoscope bell.

In a further embodiment of the invention the elastic clamping means have springs associated with the surfaces that ensure freedom from leaks. Such springs serve the purpose of maintaining elasticity, even if the elastic means are ageing.

In a further embodiment of the invention the elastic clamping means are fitted to an interchangeable part of the unit, corresponding to each type of stethoscope it is desired to interface with. This enables an interface unit to be used with a wide range of stethoscopes, provided an adapter is used to create the freedom from leaks in the interface. Such an adapter must not add an appreciable amount of dead air volume, however, unless there is feedback control of the bass frequency response.

The invention will be described in greater detail in the following with reference to the drawing, in which
Fig. 1 shows a schematic block diagram of an audio interface system according to the invention,
Fig. 2 shows a schematic block diagram of an audio interface system when integrated into an interactive computer in one or more networks
Fig. 3 is a drawing of an audio interface unit for the interface system according to the invention
Fig. 4 is a drawing of the audio interface unit cooperating with a conventional stethoscope,
Fig. 5 shows a block diagram of the audio interface unit cooperating with an electronic stethoscope,
Fig. 6 shows the sealed but releasable connection between the electronic transducer unit and the chestpiece of a stethoscope.
Fig. 7 shows the simulated influence from an acoustic leak in the coupling between the interface speaker and the stethoscope transducer.

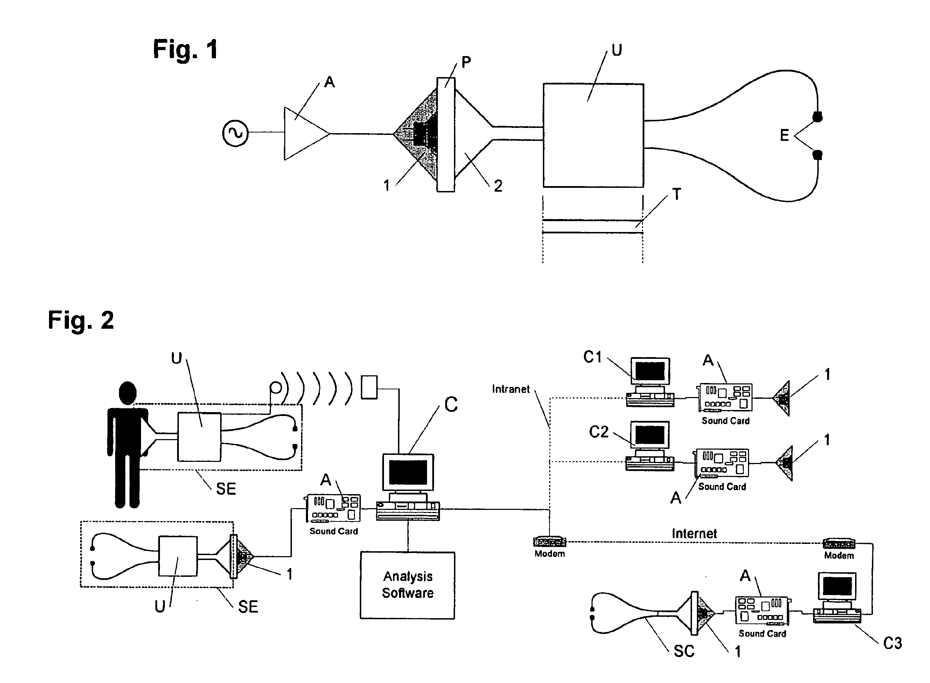

In Fig. 1 is seen an audio interface system consisting of an amplifier A, an electromagnetical driver 1, packing material P, the bell of a stethoscope 2, the earpieces E of a stethoscope in conjunction with ears indicated schematically. The packing material P is shown generalised as a material which will ensure hermetic closure between the driver 1 in the audio interface unit and the bell 2 of the stethoscope. The driver is preferably an electrodynamic driver, but any motor capable of sufficient volume transport at near DC frequencies will function. Between the bell 2 and the earpieces E there may either be an electronic unit U, preferably with digital signal processing receiving sound from the bell by means of a microphone and outputting signal processed audio signal to the earpieces E, or there may simply be a piece of tubing T. In the first case we have an electronic, preferably digital stethoscope SE, in the second case we have a conventional stethoscope SC. The audio interface system will provide an audio output at the earpieces E when the amplifier A is supplied with an electronic signal. In case the amplifier is part of a soundcard in a computer, the input will be digital from the internal data bus. In case the amplifier is connected to a telephone or a dictaphone, the input signal will be analogue.

In Fig. 2 is shown how the audio interface system is incorporated in a medical signal management system. The inputting electronic stethoscope SE provides an input to the ears of the auscultating physician, and the sounds identified (possibly extracted from temporary storage in the electronic unit U) in the stethoscope are transmitted via a wireless link to a computer C, such as a personal computer. This computer also has modems for communicating with an intranet and the Internet, it has analysis soft- and firmware, and it has a soundcard A. The soundcard is connected to the audio interface unit 1 which is joined to an electronic stethoscope used for listening to the output of the computer. Via the computers Cl, C2, or C3 other sounds may be obtained via the local intranet or the Internet. On the remote location of the computer C3 it is possible to listen by means of a conventional stethoscope SC to the sounds in C originating in the first electronic stethoscope SE mentioned above. The detailled functioning will be given in Example 1 below.

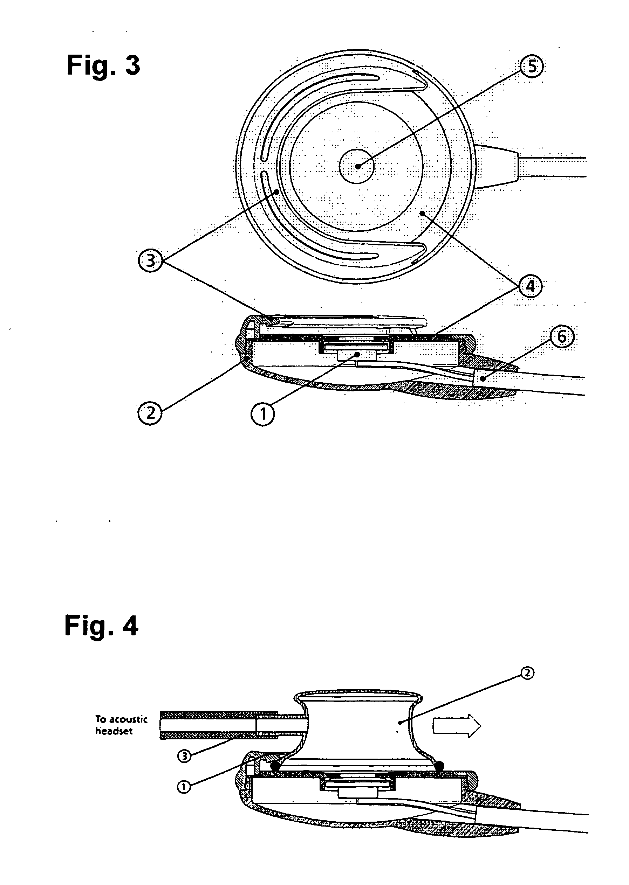

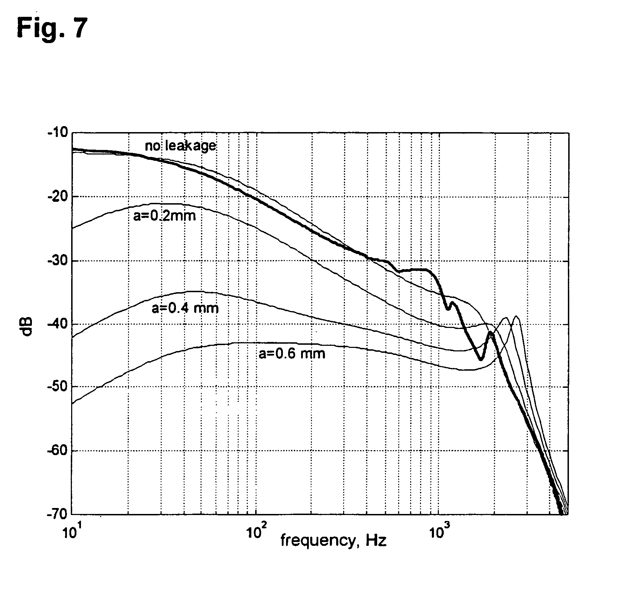

The audio interface unit shown in Fig. 3 contains a speaker 1 held by the interface housing 2. The slightly elastic lock 3 is intended for the secure and hermetically sealing application of a stethoscope chestpiece onto the audio interface mounting plate 4. This way a closed acoustic reproduction system, adequate for reproduction of very low frequency signals - principally down to static pressures - is completed as the speaker 1 transmits acoustic signals through the opening 5. The speaker 1 is electrically connected to the signal source by means of the wire 6. Figure 7 demonstrates the effect of introducing a tiny leak in the coupling between the speaker and the stethoscope bell: The figure clearly demonstrates how a leak shaped as an acoustic slit of size up to 0.6x1x1 mm (height, width and length along the direction of sound propagation) potentially reduces the resulting sound pressure level by more than 20 dB at 100 Hz and even more at lower frequencies. Curves are shown in thin lines for slit heights a = 0.0mm, 0.2mm, 0.4mm, and 0.6 mm. This demonstration stresses the importance of ensuring adequately controlled acoustic sealing application of the stethoscope (e.g.) bell to the reproduction speaker.

In cooperation with a conventional stethoscope, as shown in Fig. 4, the audio interface lock 1 provides physical fixation between the interface and the stethoscope chestpiece 2. The closed acoustic reproduction system now consists of the entire internal stethoscope volume from the chestpiece 2 through the tube 3 to the eartips 4 which are hermetically sealing to the listener's ears.

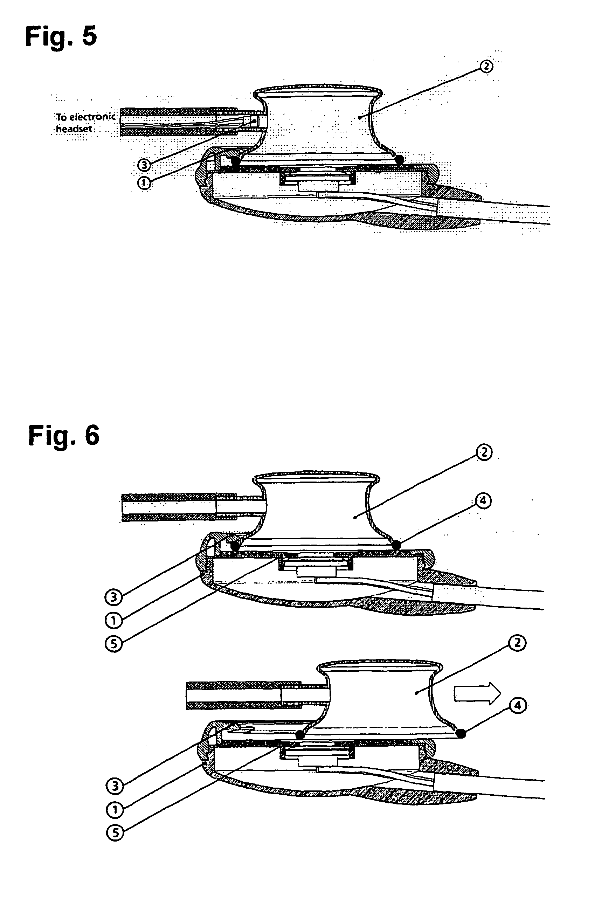

In cooperation with an electronic stethoscope, as shown in Fig. 5, the audio interface lock 1 provides physical interlocking between the interface and the stethoscope chestpiece 2 holding the input transducer of the electronic stethoscope. The closed acoustic reproduction system now consists of the narrow coupling volume between the audio interface and the chestpiece input transducer interior and microphone 3 regardless of the remaining stethoscope system.

The illustration in Fig. 6 shows the simple release of the connection between the audio interface 1 and the stethoscope chestpiece 2. In this preferred embodiment of the audio interface the fixation is maintained by the lock 3 acting as a stretched spring pressing on the upper part of the chestpiece rim 4 thereby creating a downward force connecting the chestpiece to the audio interface. The lock being shaped as a fork allows for easy release by horizontally sliding the two items apart along the mounting plane surface 5 through the opening in the fork shaped lock 3.

The invention will be further described by means of the following

Example 1. The interactive use of an electronic stethoscope.

A sound is recorded via an electronic stethoscope and a data link to a PC (Fig. 2). Here time-frequency signal processing is performed, and certain features are enhanced. The sound is returned to a physician's conventional stethoscope by means of the audio interface system according to the invention.

An auscultation signal is picked up and digitised into a fixed 16 bit dynamic range by the use of a digital/electronic stethoscope. Prior to digitisation the analogue signal is pre-emphasised, by subjecting it to time-differentiation, in order to optimise the signal to noise ratio of the digitally represented signal. From the digital storage of the stethoscope the signal now transfers without loss to the memory of a personal computer using a cord-less data link, i.e. an infrared link such as e.g. the IrDA system or a radio frequency (RF) link such as e.g. the Bluetooth system. Through exportation of the signal to a personal computer the signal may be archived in a database or patient file for later inspection. Furthermore the handling on a personal computer enables specialised signal analysis that it would not be relevant to include on the stethoscope itself.

One interesting external signal analysis operation could be to perform a special type of windowing on a cardiac signal thereby enabling separate playback of e.g. the isolated cardiac systole sound. By cutting out e.g. the systole and thereby separating this segment from the dominating first and second characteristic heart sounds perceptual masking effects arising from these may be minimised, and the physician will more easily be able to hear the delicate details of a systolic or diastolic murmur.

Once the signal analysis is complete, the output signal must be presented to the physician for examination by means of a stethoscope or a similar acoustic reproduction system. The typical personal computer headsets are far from adequate in this context as they normally fail to reproduce the very low frequencies down to approximately 10 Hz, which are essential for the description of the human cardiac sound. Instead the output signal may be transferred back to the storage on the digital/electronic stethoscope, using the infrared data link, for later audible presentation to the physician.

Alternatively the signal may be presented using the invention in conjunction with either an electronic or a conventional stethoscope as illustrated on Figs. 2-5. In this situation the audio interface according to the invention is driven via e.g. the headset output terminal on personal computer sound card interface. Typically such sound cards may deliver output voltages up to 2 V RMS which is adequate for the reproduction of cardiac signals at amplitudes higher than 120 dB SPL.

During reproduction from the digital personal computer using the sound card and the audio interface according to the invention, the limited dynamic range of the sound card may degrade the signal to noise ratio of the reproduced acoustic signal thereby adding significant amounts of e.g. quantization noise to the signal. Especially noise in the middle frequency region, approximately from 900 Hz to 6000 Hz where the normal functioning ear has highest sensitivity, causes degraded sound quality as it potentially masks delicate elements of the cardiac sound signal. To minimise noise from the digital output stage the primary correction for the signal pre-emphasis is realised for in the analogue domain directly at the audio interface speaker using a first order low-pass filter. Any necessary residual correction, ensuring desired frequency characteristic of the audio interface, is carried out using digital filtering on the personal computer. By properly combining the choice of reproduction stethoscope and residual digital correction filtering, the frequency characteristic of the total reproduction system may be controlled.

Instead of enabling reproduction of the sound signal privately and solely for the same physician who originally recorded and analysed the sound signal, the reproduction could take place at a physically distant site, e.g. at the site of another physician providing a second opinion to the diagnosis. For this purpose the digital domain sound signal may be sent loss-free anywhere, e.g. using the Internet, an intranet or a similar cable system, or a cordless transmission system. At the recipient the digital domain signal may be subjected to further analysis or merely reproduced using the recipient's personal computer in combination with the audio interface coupled to his personal stethoscope.

Alternatively the signal could be distributed simultaneously to a multitude of recipients, physically positioned locally or distant. For example, for the education of future physicians where a lecturer demonstrates the use and benefit of the stethoscope, he would take advantage of the capability for loss-free distribution of the digital domain stethoscope sound signal to each student separately, who then reproduces the signal using a local personal computer in combination with the audio interface and his own private stethoscope. The scenario would allow the student to obtain a high quality reproduction of the stethoscope sound signals and at the same time permit his use of his own personal stethoscope as a reproduction medium, thereby enabling stronger familiarisation between the student and his choice of personal stethoscope.

In relation to the ability of the audio interface to be highly flexible towards a multitude of different stethoscope types, one mechanical solution (discussed below) suggests the presence in the audio interface mechanical system of an exchangeable member having the property of customising the interface to a specific stethoscope type. This way the audio interface may be adapted to any given type of stethoscope simply through the exchange of the central mechanical member. Since, however, the proper function of the audio interface only occurs when it, in combination with the stethoscope, offers a total reproduction system with known acoustic properties, including the frequency characteristic from the electrical input to the acoustic output at the listener's ears, a tuning of the residual digital filtering may be required. For this purpose the specific exchangeable mechanical member is identified through a type of code which is printed on its structure, easily readable for the user, who will enable the adequate digital filtering automatically through entering this code into a dedicated software system controlling the reproduction on the personal computer. The system, including the mechanical elements as well as the software elements enables, in principle, an infinite number of combinations of an audio interface and a stethoscope, furthermore it ensures that the desired acoustic properties of the system are present for every combination.

A key feature of the mechanical design of an audio interface according to the invention is the ability to ensure a secure and sealed fixation onto a stethoscope when mounted and at the same time enable for a quick attachment or release respectively. Furthermore these properties of the audio interface should be flexible towards a multitude of different stethoscope chestpiece shapes and geometries thereby allowing for use in combination with many different stethoscopes, principally all known types.

In addition to the above exemplified signal analysis scheme it can be relevant to the interpretation of the auscultation sound signals to perform different kinds of combined audible and visual presentations of the signal. E.g. in a time/frequency plot it is possible to place cursors both in time and frequency domain and thereby extract features visible on the plot. These features can be replayed for perception and education. In another approach time cursors can be placed on the time signal to measure time length of systole, diastole and the heartbeats. These measurements are used to calculate systole/diastole ratio, heart rate and arrhythmia parameters. In yet another approach automated algorithms finding systole and diastole in heart sounds can be audited visually before a murmur finding algorithm is initiated. Murmurs will be visualized on the screen and can be replayed for higher perception, audit and educational purposes. Based on murmur information and other objective patient information an expert system is able to predict possible diseases.

The mechanical construction of the electroacoustic transducer is documented in

### Example 2.

The preferred mechanical design of the audio interface and its interlock, as illustrated in Fig. 3 and Fig. 6, allows for mounting on at least three different known types of stethoscope, of which one is a conventional acoustic type and two are electronic stethoscope types. Common to all three is that they employ a very similar geometry on the lower part of their respective chestpieces, at the site of interlock with the audio interface, whereby they have the approximately same profile in these lower extremities. The special geometry is characterised primarily by the presence of a waistline, a local diameter reduction of certain dimensions, especially its diameter and elevation over the plane of the stethoscope chestpiece lower extremity where it interconnects to the audio interface. This waistline allows for the application of vertical downward forces pressing the stethoscope chestpiece towards the audio interface thereby establishing a fixation between the two objects. The assembly is illustrated in Fig. 4 where the upper figure shows the secure and sealed fixation of the stethoscope chestpiece upon the audio interface where the lock-spring 3 applies downward force on the chestpiece rim 4 over at least 180° of the circular chestpiece rim. During assembly or release of the two objects slide horisontally through the opening in the lock-spring 3 as shown in Fig. 3.

As it is crucial for the proper functioning of the audio interface that the connection to the stethoscope chestpiece is hermetically sealed to an extent whereby the transmission characteristic below 10 Hz is not influenced, the seal along the chestpiece rim and the audio interface mounting plane surface is critically important. To ensure sufficient sealing, the application force acting between the chestpiece and the audio interface should be large and either or both of the chest piece rim and the mounting plane surface should be slightly resilient whereby they would mutually adapt their shape, thereby minimising leakage. In case a soft, e.g. rubber layer, is cast on top of the mounting plane the surface should preferably be coated with a suitable lubricant, thereby enabling easy sliding of the chestpiece rim across the mounting plane surface. A practical measurement on a seal obtained between an audio interface according to the invention and a stethoscope chestpiece as shown in Fig. 4 is shown as a heavy line curve in Fig. 7. It will be noted that there is full accord with the ideal curve for the extreme bass frequencies.

In order for the audio interface to allow more flexibly for a larger multitude of different chestpiece geometries, the audio interface may preferably be designed so as to allow for a simple exchange of the lock-spring 3 as shown in Fig. 3. Thereby it is possible to have stethoscope-specific lock-springs, and all that it is required for a physician to make use of the audio interface, is for him to attach the proper lock-spring on the interface housing and mount the audio interface on his specific stethoscope chestpiece.

Another method of assembly and fixation of the audio interface onto a stethoscope chestpiece may be via a type press-and-turn mechanism where the chestpiece, when brought into contact with the audio interface mounting plane, through a turning movement in the plane of the mounting plane surface of the two objects against each other, causes the two objects to interlock to a secure and sealed connection. The fixation would be enabled by means of a third mechanical member having the property of enabling change of inner diameter when subjected to that specific turning movement, of the audio interface against the chestpiece, whereby this member effectively applies force on the outer periphery or rim of the inserted chestpiece thereby ensuring fixation. To some extent this application force would preferably be orientated downwards toward the audio interface mounting plane, thereby more easily enabling a secure and sealing assembly.

## Claims

1. An audio interface system for medical use comprising an electronic source providing an electronic representation for audio signals, and a signal path for audio signals comprising a headset connectable to the ears of a physician or other medical personnel, **characterised in that** said electronic source is connected to an electroacoustic transducer which is releasably connected to the chestpiece of a stethoscope in a sealed manner in order that the earpieces of the stethoscope convey the audio signals to the ear.

2. An audio interface system according to claim 1,
**characterised in that** the signal path for the audio signals obtained in the chestpiece is constituted by the tubing and earpieces of a conventional stethoscope.

3. An audio interface system according to claim 1,
**characterised in that** the chestpiece is constituted by a microphone providing an input to signal processing means in or in conjunction with the stethoscope which provides an output signal to electroacoustic transducers in conjunction with the earpieces of a stethoscope.

4. An audio interface system according to claim 1,
**characterised in that** the electronic representation of the audio signals has been signal processed in order to compensate for the transfer function of the stethoscope.

5. An audio interface system according to claim 3,
**characterised in that** the signal processing comprises a linearising compensation of the transfer characteristics of the electroacoustic transducer connected to the chestpiece.

6. An audio interface system according to claim 5,
**characterised in that** the linearising compensation is obtained by feedback via a monitoring microphone mounted in conjunction with the coupling cavity.

7. An audio interface system according to claim 1,
**characterised in that** the electronic source providing an electronic representation for audio signals is a soundcard connected to a personal computer.

8. An audio interface system according to claim 1,
**characterised in that** the electronic source for audio signals is a receiver for telephone signals.

9. A use of an audio interface system according to claim 1 on a specific audio signal, **characterised in that** the audio signal is a representation of physiological signals mixed with noise signals obtained from the environment.

10. A use of an audio interface system according to claim 9 on a physiological signal, **characterised in that** the physiological signal is a standardised physiological signal for ausculation.

11. An audio interface system according to claim 1, **characterised in that** the releasable connection comprises elastic clamping means.

12. An audio interface system according to claim 11,
**characterised in that** the elastic clamping means have springs associated with the surfaces that ensure tightness.

13. An audio interface system according to claim 11,
**characterised in that** the elastic clamping means are fitted to an interchangeable part of the unit, corresponding to each type of stethoscope it is desired to interface with.

14. A use of an audio interface system according to claim 9 for recording and reproducing signals to be utilised by means of a stethoscope, optionally comprising signal processing and/or feature extraction means, **characterised in that** the recording occurs via an electronic output from an electronic stethoscope and the reproduction occurs via an interface according to claim 1.

## Patentansprüche

1. Ein Tonfrequenzschnittstellensystem für medizinische Anwendung, umfassend eine elektronische Quelle, die eine elektronische Darstellung von Tonsignalen gibt, sowie einen Signalweg für Tonsignale, den einen Kopfhörer umfasst, den zu den Ohren eines Artzes oder anderes medizinisches Personal verbunden werden kann, **dadurch gekennzeichnet**, das die genannte elektronische Quelle an einer elektroakustischen Wandler verbunden ist, den lösbar auf versiegelter Weise zu einem Bruststück eines Stethoskops verbunden werden kann, damit die Ohrstücken des Stethoskops die Tonsignale an die Ohr leiten.

2. Ein Tonfrequenzschnittstellensystem gemäss Anspruch 1, **dadurch gekennzeichnet, dass** der Signalweg für die in dem Bruststück erhaltenen Tonsignale von den Schläuchen und Ohrstücken eines konventionellen Stethoskops gestaltet ist.

3. Ein Tonfrequenzschnittstellensystem gemäss Anspruch 1, **dadurch gekennzeichnet, dass** der Bruststück als ein Mikrofon, das ein Eingangssignal zu Signalverarbeitungsmittel in oder in Verbindung mit dem Stethoskop, welches ein Ausgangssignal zu elektroakustischen Wandlern, die mit den Ohrstücken eines Stethoskops verknüpft sind, gestaltet ist.

4. Ein Tonfrequenzschnittstellensystem gemäss Anspruch 1, **dadurch gekennzeichnet, dass** die elektronische Darstellung der Tonsignale einer Signalverarbeitung ausgesetzt worden ist, um die Übertragungsfunktion des Stethoskops zu kompensieren.

5. Ein Tonfrequenzschnittstellensystem gemäss Anspruch 3, **dadurch gekennzeichnet, dass** die Signalverarbeitung eine linearisierende Kompensation der Übertragungscharacteristiken des zu dem Bruststück verbundenen elektroakustischen Wandlers umfasst.

6. Ein Tonfrequenzschnittstellensystem gemäss Anspruch 5, **dadurch gekennzeichnet, dass** die linearisierende Kompensation durch Rückkupplung mittels eines Überwachungsmikrofon, das in Verbindung mit dem Kupplungshohlraum montiert ist, erreicht wird.

7. Ein Tonfrequenzschnittstellensystem gemäss Anspruch 1, **dadurch gekennzeichnet, dass** die elektronische Quelle, die eine elektronische Darstellung von Tonsignalen gibt, eine zu einem Personalcomputer verbundene Soundkarte ist.

8. Ein Tonfrequenzschnittsteltensystem gemäss Anspruch 1, **dadurch gekennzeichnet, dass** die elektronische Quelle für Tonsignale eine Telephonkapsel ist.

9. Anwendung eines Tonfrequenzschnittstellensystem gemäss Anspruch 1 für ein bestimmtes Tonsignal, **dadurch gekennzeichnet, dass** das Tonsignal ein physiologisches Signals gemischt mit Geräuschsignale aus der Umgebung darstellt.

10. Anwendung eines Tonfrequenzschnittstellensystem gemäss Anspruch 9 für ein physiologisches Signal, **dadurch gekennzeichnet, dass** das physiologische Signal ein standardisiertes physiologisches Signal für Auskultation ist.

11. Ein Tonfrequenzschnittsiellensystem gemäss Anspruch 1, **dadurch gekennzeichnet, dass** die lösbare Verbindung elastische Spannmittel umfasst.

12. Ein Tonfrequenzschnittstellensystem gemäss Anspruch 11, **dadurch gekennzeichnet, dass** die elastische Spannmittel Feder umfassen, die mit denjenigen Oberflächen, die Dichtung sichern, verknüpft sind.

13. Ein Tonfrequenzschnittstellensystem gemäss Anspruch 11, **dadurch gekennzeichnet, dass** die elastische Spannmittel an einer auswechselbaren Teil des Einheits verbunden ist, die zu den jeweils gegebenen Typen von Stethoskop, zu welchem eine Schnittstelle gewünscht wird, passt.

14. Anwendung eines Tonfrequenzschnittstellensystem gemäss Anspruch 9 für die Aufnahme und Wiedergabe von Signale, die von einem Stethoskop ausgenutzt werden sollen, wahlweise Signalverarbeitungs- und/oder Signaleigenschaftanalysiermittel umfassend, **dadurch gekennzeichnet, dass** die Aufnahme mittels eines elektronischen Ausgangs von einem elektronischen Stethoskop und die Wiedergabe mittels eines Schnittstellensystems gemäss Anspruch 1 vor sich gehen.

## Revendications

1. Un système d'interface audio pour utilissage médicale comprenant une source électronique fournissant une représentation électronique des signaux audio, et un chemin de signaux audio comprenant des écouteurs qui pouvent être connectées aux oreilles d'un médecin ou autre personne médicale, **caractérisé en ce que** ledit source électronique est joint a un transducteur électroacoustique étant joint dans une manière étanche mais déclencheable a une pièce thoracique d'un stéthoscope afin que les tubes écouteurs du stéthoscope portent les signaux audio aux oreilles.

2. Un système d'interface audio selon revendication 1, **caractérisé en ce que** le chemin de signaux audio obtenu dans la pièce thoracique est constitué par les tubes et écouteurs d'un stéthoscope conventionel.

3. Un système d'interface audio selon revendication 1, **caractérisé en ce que** la pièce thoracique est constituée par un microphone qui fournit un signal d'entrée aux moins de traitement de signaux dans ou en corresponcence avec le stéthoscope qui fournit des signaux de sortie au transducteurs électroacoustiques en conjonction aux écouteurs d'un stéthoscope.

4. Un système d'interface audio selon revendication 1, **caractérisé en ce que** le representation électronique des signaux audio a éte traité afin que compenser le fonction de transfert du stéthoscope.

5. Un système d'interface audio selon revendication 3, **caractérisé en ce que** le traitement de signal comprend une compensation linéarisant du caractéristique de transfert du transducteur electroacoustique lié a la pièce thoracique.

6. Un système d'interface audio selon revendication 5, **caractérisé en ce que** la compensation linéarisant est obtenu par contreréaction par un microphone-moniteur monté en conjonction du cavité de couplage.

7. Un système d'interface audio selon revendication 1, **caractérisé en ce que** la source électronique fournissant une represantation électronique de signaux audio est une carte son reliée a un ordinateur personel.

8. Un système d'interface audio selon revendication 1, **caractérisé en ce que** la source électronique de signaux audio est un recepteur de signaux téléphoniques.

9. L'utilisation d'un système d'interface audio selon revendication 1 pour un signal audio specifique, **caractérisé en ce que** le signal audio est une représentation de signaux physiologiques mêlé a de signaux de bruit obtenus de l'environnement.

10. L'utilisation d'un système d'interface audio selon revendication 1 pour un signal physiologique, **caractérisé en ce que** le signal physiologique est un signal physiologique standardisé pour auscultation

11. Un système d'interface audio selon revendication 1, **caractérisé en ce que** le joint déclencheable comprend un dispositif de serrage élastique.

12. Un système d'interface audio selon revendication 11, **caractérisé en ce que** le dispositif de serrage élastique comprend des ressorts lié aux surfaces assurant la étancheitée.

13. Un système d'interface audio selon revendication 11, **caractérisé en ce que** le dispositif de serrage élastique est monté a une partie éxchangeable de l'unité, en correspondence au chaque type de stéthoscope, avec lequelles on desire faire de interface.

14. L'utilisation d'un système d'interface audio selon revendication 9 pour enregistrer et reproduir des signaux à utiliser par un stéthoscope, facultativement comprenant traitement de signal et/ou moins d'éxtraction de caractéristiques, **caractérisé en ce que** l'enregistrement a lieu par une sortie électronique d'un stéthoscope électronique, et la reproduction a lieu par un interface selon revendication 1.
